Europäisches Patentamt

European Patent Office

Office européen des brevets

(11) Publication number: **0 284 258 B1**

# EUROPEAN PATENT SPECIFICATION

(45) Date of publication of patent specification: **19.11.92**  (51) Int. Cl.5: **A61D  7/00, A61M 31/00**

(21) Application number: **88302148.7**

(22) Date of filing: **11.03.88**

(54) **Bolus.**

(30) Priority: **12.03.87 GB 8705825**

(43) Date of publication of application:
**28.09.88 Bulletin  88/39**

(45) Publication of the grant of the patent:
**19.11.92 Bulletin  92/47**

(84) Designated Contracting States:
**DE FR GB NL**

(56) References cited:
**EP-A- 0 062 391**
**GB-A- 2 186 291**
**US-A- 4 684 516**

(73) Proprietor: **CASTEX PRODUCTS LIMITED**
**Woodside Works Woodside Street New Mills**
**Stockport, Cheshire SK12 3HG(GB)**

(72) Inventor: **Whitehead, Derek James**
**103 Dickens Lane**
**Poynton Cheshire(GB)**

(74) Representative: **Barker, Rosemary Anne et al**
**c/o Mewburn Ellis, 2 Cursitor Street**
**London EC4A 1BO(GB)**

Rank Xerox (UK) Business Services

**Description**

This invention concerns a bolus or pellet for use in the administration of trace elements and/or therapeutic and/or other biologically active agents or materials to ruminants, such as cattle and sheep.

It is already known, for instance, to construct such a bolus as a cylindrical or other elongate body of a material to be dosed to an animal, (for example magnesium, or magnesium alloy where an animal is required to be treated for hypomagnesaemia), this body may be externally coated, for instance, to ensure that, when it is present within the dosed animal's rumeno-reticular sac rumen liquid can have access to the magnesium, to degrade or dissolve the same, only at predetermined areas for instance at one or both ends of the body.

Furthermore it is already known to form the body so as to be hollow and contain a filling of a therapeutic or other agent which is release to the animal with progressive degradation of the body. The arrangement may be such that the filling is substantially continuous (e.g. being a mass compressed into the casing) in which case continuous dosing of the animal with the agent is achieved. Proposals are also known in which the filling is composed of a succession of tablets or pellets, which may be of the same or different materials, which are separate from one another, so that successive or pulsed doses are released within the animal's rumeno-reticular sac as the bolus is gradually eroded away. In such a pulsed-release device the body may comprise a plurality of segments which fit to one another and define respective cavities for accommodating respective tablets or discs of or including active material(s). Experience shows that the presence of iron or copper in electrically-conducting juxtaposition to the magnesium of the bolus influences the rate at which the magnesium is eroded away by reason of the galvanic effects occurring when the iron/copper and magnesium are exposed to the rumen juices.

In the design of boluses of the kind with which the present invention is concerned, it is appropriate to provide for the overall density of the device to be in excess of 2.25 gm/ml, so as to minimise the possibility of expulsion of the device, e.g. by regurgitation, and for this purpose proposals have been made to construct the bolus with a steel or iron end weight, to form part of the bolus with a hollow chamber to accommodate a filling of iron shot or the like, or to incorporate iron shot into the active material which is released by the bolus. Where there is an iron or steel end weight in contact with the magnesium or magnesium alloy so that there is electrical continuity therebetween the galvanic effect above referred to is obtained, but where use is made of iron shot, the shot is usually coated with iron oxide so even when closely packed it forms an essentially non-conductive matrix. End weight constructions including iron shot held in hollow chambers and dispersed through a degradable magnesium tube are described in EP0164927. A nonconductive matrix is often formed where the shot is admixed with a therapeutic agent and compressed into the hollow body, since the therapeutic material is in most instances likely to be non-conductive. For this reason, we have already proposed, in a prior co-pending application for patent the incorporation of a specifically electrically conductive material such as powdered graphite, in order to ensure the achievement of the galvanic effects above discussed.

It is also known to form pellets or boluses of iron powder compacted with, for example, selenium powder or cobalt oxide. Such pellets are hard and virtually insoluble in the rumen juices and it is therefore necessary that each animal given such a pellet should also be given an accompanying 'grinder' pellet to ensure that the pellet containing the active ingredient is continually abraded to liberate the cobalt or selenium. Such pellets suffer from the disadvantages that they are inefficient, as a large excess of the trace element is given to the animal only a part of which may be used and the dosage rate is neither very exact nor reproducible. In use such pellets tend to become coated with insoluble calcium compounds which retard dissolution.

A further form of known pellet comprises copper, cobalt and selenium dissolved in a sparingly soluble glass matrix. This pellet also has the tendency to become coated with isoluble compounds thus reducing the degradation rate.

An object of the present invention is to provide a pellet or bolus which includes a component which functions effectively to increase the overall density of the device to minimise the risk of its expulsion, this component also functioning as an abrasive to case degradation of a second pellet or bolus.

With this object in view the present invention provides a bolus or pellet for use in the administration of one or more active agents to a ruminant, said bolus or pellet including a body consisting of a particulate weighting material characterised in that said weighting material is mixed with one or more biologically active agents, and in that substantially all the weighting material particles have a roughened, spiky or uneven surface and are dispersed together with the active agents in a binder material such that the body's exterior surface is uneven and acts as an abrasive.

A preferred composition comprises from 60% to 90% by weight of weighting material, 5% to 30% by weight of biologically active agent and 5% to 15% by weight of binder material.

Advantageously a water soluble agent is provided in the binder such that degradation of the body by abrasion is assisted by an amount of dissolution.

Advantageously the binder is a wax, for example parafin wax or beeswax. Other binders such as sulphur or plastics may also be used. Alternatively the binder may comprise or include a soap such as sodium oleate, sodium palumitate or sodium stearate to provide the binder with limited water solubility.

Optionally the body can be enclosed in a hollow body of magnesium or magnesium alloy.

Iron or steel grit may be used as the weighting material and may be produced by crushing shot, e.g. spherical shot particles. The grit so produced is acicular in form and generally free from superficial oxide. Consequently addition of graphite is unnecessary to achieve an electrically conductive matrix when the grit is compacted with active drug(s) and/or other material(s) desired to be dosed to the animal, and with binder (s).

Advantageously the bolus is formed as a cylindrical body enclosed in an insoluble and non-degradable tube-like container which is preferably formed from a plurality of closely abutting rings leaving one or both ends of the body uncovered. Preferably the rings are of plastic although metals, ceramics or other materials insoluble in the rumen juices may be used.

The invention will be described further,by way of example, with reference to the accompanying drawings, in which:-

Fig. 1 is a perspective view of a first preferred embodiment of a bolus or pellet according to the invention.

Fig. 2 is an end view of a second embodiment of the invention;

Fig. 3 is a cross sectional side elevation of a third embodiment; and

Fig. 4 is a cross sectional side elevation of a fourth embodiment of the invention.

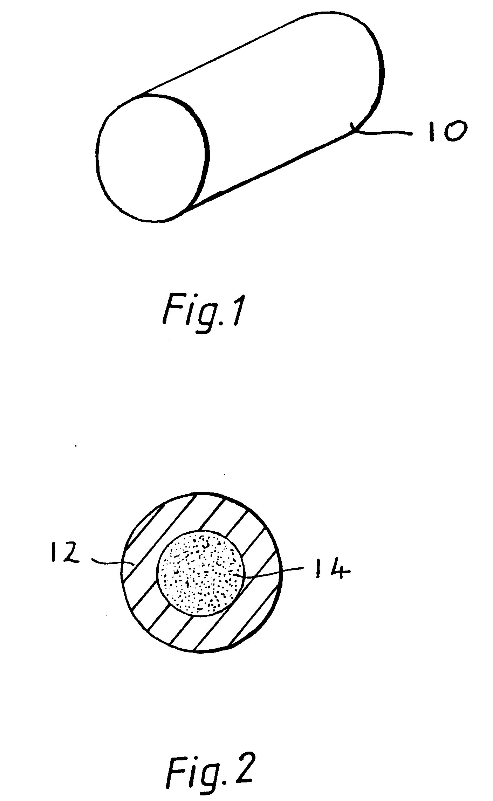

A first embodiment of the bolus or pellet of the invention is illustrated in Fig. 1 in which a pellet 10 is constructed as a compact body formed of a matrix of wax, in which an active agent is dispersed evenly, and which holds together a close packed steel grit. The wax used may be paraffin wax and the steel grit may be G07 grade, i.e. 0.18 mm - 0.42 mm mesh size although other waxes and grades of steel or iron grit may be employed. The steel grit is acicular in shape and has a roughened, spiky or uneven surface providing sharp edges. Such pellets 10 are normally given in pairs and the action of the sharp edged grit on the softer matrix results in a uniform rate of degradation of the pellets by abrasion. It is therefore important that the weighting material used should be able to function as an abrasive and have generally uneven surfaces. The rate of degradation of the pellets 10 may be controlled by varying the size and concentration of steel grit in the pellet and by using waxes of different hardness and melting point. The higher the proportion of grit used and the harder the wax, the larger the life of the pellet. The relatively low packing density of fine steel grit allows a comparatively large volume of wax binder and contained active matter to be used whilst retaining high hardenss and mechanical strength of the pellet.

Example 1

| Paraffin wax, as binder (melting point 52°C) | 8.5% |
| Benzimidazole, as active agent | 6.1% |
| Steel grit, grade G07 | 85.4% |
| Pellet Density | 4.2 gm/ml |

In example 1 the paraffin wax is melted and the anthelmintic Benzimidazole stirred in to produce a uniform creamy liquid. The G07 grit is preheated to 60°C and is then added to the liquid and continously stirred while the mixture is allowed to cool. A uniform, relatively free flowing, coarse powder is obtained which can be moulded in a hardened steel die at a pressure of approximately 10 tons to form a cylindrical pellet of 25 mm diameter and 25 mm length. Two such pellets are administered to an animal as desired and the action of the pellets on each other cause uniform degradation, liberating the anthelmintic over a period of several weeks.

The foregoing example suffers from the disadvantage that although the rate of erosion per unit surface area remains approximately constant the rate of drug release decreases as the pellets get smaller. It is possible to form a composite pellet where this disadvantage is partially overcome by producing pellets in a similar manner to that previously described wherein the centre portion of the pellet have a higher active agent content than the ends or exterior zones. The mouldability of wax powders makes such pellets easy to manufacture.

Example 2

| Paraffin wax as binder | 6.8% |
| Sodium oleate | 3.7% |
| Copper oxide | 25.5% |
| Cobalt oxide | 0.25% |
| Selenium | 0.2% |
| Steel grit, G07 grade | 63.55% |

In this example the wax is melted and mixed with the other ingredients to produce a uniform paste. The mixture is cooled and reduced to a coarse powder. Compressing 50 gm of such powder in a 25 mm diameter pellet mould at a pressure of 10 tons produces a hard pellet with a density of 4 gm/ml. Two such pellets provide sufficient copper, cobalt and selenium for a full grown cow for 200 days.

The compacted powder of Example 2 is electrically conductive with negligible resistivity and hence may be used to form the core electrode of a dual purpose bolus used to supply magnesium and trace elements such as copper, cobalt and selenium to ruminants. A suitable configuration for such a bolus is shown in Fig. 2 and comprises a tubular magnesium alloy body 12, 90 mm long x 25 mm diameter x 13 mm internal diameter which is die cast from an alloy containing 12% aluminium, 2% copper and 86% magnesium. A central core 14 of active ingredient according to example 2 comprises 45 gm of mixture pressed in situ into the tube. Two such devices administered to a cow provide sufficient trace elements over the lifespan of the bolus which is usually several months.

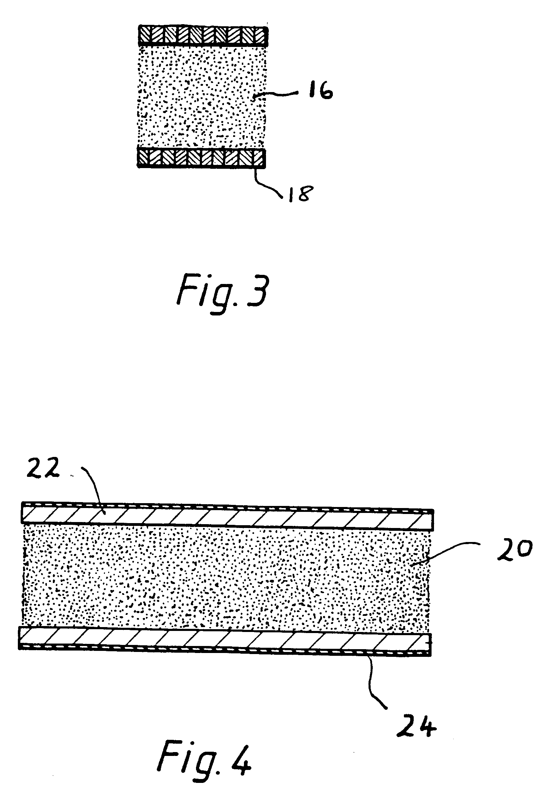

In a third embodiment shown in Fig. 3 a core filling 16 comprises:-

Example 3

| Paraffin Wax | 4.6% |
| Sodium Oleate | 1.9% |
| Sodium Palmitate | 1.5% |
| Sodium Stearate | 0.5% |
| Benzimidazole | 9.8% |
| Steel grit G07 grade | 81.7% |

These ingredients are formed into a powder as previously described in Example 2. A bolus casing or hollow body is formed by placing 10 rigid P.V.C. rings 18 into a hardened steel die so that they abut closely. Each ring has an external diameter of 25 mm, width 2 mm and internal diameter 21.5 mm. 40 gms of the powder are introduced into the die and are pressed with a load of 8 tonnes to form a compacted core 16 surrounded by the rings 18.

Two such pellets given to a cow will degrade in a uniform manner over a period of about 120 days, the rings being operative to limit the access of the rumen contents to the bolus exterior except at the respective open ends. The rings 18 are successively discarded as the core 16 is degraded or eroded. The discarded plastic rings are expelled by the animal as their density is insufficient to permit them to remain in the rumen. The bolus as a whole has a density of 3.5 gm/ml and the compacted powder is electrically conductive. Thus the core then formed may also be used with a magnesium tube as shown in Fig. 2.

A further embodiment of the invention is illustrated in Fig. 4 and uses the following example of a filling 20 suitable for use in boluses of the kind comprising an external tubular body or shell 22 of magnesium alloy. externally protected against contact by rumen fluids except at its ends by a coating 24 so that degradation occurs only from the ends of the bolus for continous release of the magnesium of the body and the active material of the filling, the percentages being by weight:-

Example 4

4

| Benzimidazole, as active agent | 8.4% |
|---|---|
| Sucrose, as binder | 12.6% |
| Steel grit, grade G07 | 78.0% |
| Zinc stearate, as lubricant | 1.0% |

Appropriately compressed, for example in a 25 mm diameter die, at a load of 5 tonnes, one obtains a compacted filling 20 having a density of about 4.5 gm/ml, and this may be filled into the body or shell 22 by forcing it therein by means of a press, if desired as a succession of tablets.

The bolus requires no further weighting and will normally be retained in the dosed animal's rumeno-reticular sac without the need for further weighting, and naturally the steel grit, produced for instance by crushing spherical steel shot, provides the appropriate electrical continuity to ensure controlled degradation of the bolus.

It will be appreciated from the foregoing examples that a preferred composition for forming a bolus or pellet according to the invention comprises from 60 to 90% by weight of particulate weighting material and 5 to 30% by weight of biologically active agent dispersed in a matrix of 5 to 15% by weight of binder material. Where the bolus or pellet is provided with a coating or external shell the percentages do not include the weights of these additional components.

The invention is not confined to the precise details of the foregoing examples and variations may be made thereto. Thus, of course, iron grit, obtained for instance, from crushing iron shot or other iron particles may be employed in the place of the steel or another suitably dense material may be used; moreover the invention may be applied in the case where the filling within the body consists of a plurality of separate compressed tablets which may be the same as or different from each other, provided that each or appropriate ones of such tablets embody the ferrous grit conforming to the invention so as to achieve the requisite galvanic effect.

## Claims

1. A bolus or pellet for use in the administration of one or more active agents to a ruminant, said bolus or pellet including a body (10, 14, 16, 20) consisting of a particulate weighting material, characterised in that said weighting material is mixed with one or more biologically active agents, and in that substantially all the weighting material particles have a roughened, spiky or uneven surface and are dispersed together with the active agents in a binder material such that the body's exterior surface is uneven and acts as an abrasive.

2. A bolus or pellet as claimed in claim 1 wherein the binder is a wax.

3. A bolus or pellet as claimed in claim 2 wherein the binder is paraffin wax.

4. A bolus or pellet as claimed in claims 1, 2 or 3 wherein the binder includes a water soluble agent.

5. A bolus or pellet as claimed in claim 4 wherein the binder includes a soap.

6. A bolus or pellet as claimed in claim 5 wherein the binder includes sodium oleate.

7. A bolus or pellet as claimed in claim 5 wherein the binder includes sodium palmitate.

8. A bolus or pellet as claimed in claim 5 wherein the binder includes sodium stearate.

9. A bolus or pellet as claimed in any preceding claim wherein the particulate, non-uniform, weighting material is iron or steel grit.

10. A bolus or pellet as claimed in any preceding claim wherein the body is partially enclosed in a hollow body (12,18,22)

11. A bolus or pellet as claimed in claim 10 wherein the hollow body is a tube of magnesium or magnesium alloy (12,22).

**12.** A bolus or pellet as claimed in claim 10 wherein the hollow body is provided by a plurality of closely abutting rings (18).

**13.** A bolus or pellet as claimed in claim 12 wherein the rings (18) are of a material insoluble or non-degradable in rumen juices.

**14.** A bolus or pellet as claimed in claim 13 wherein the rings (18) are of plastics.

**15.** A bolus or pellet as claimed in claim 9 in which the iron or steel grit is produced by crushing spherical shot.

**16.** A bolus or pellet as claimed in claim 1 in which the body (10, 14, 16, 20) comprises from 60 to 90% by weight of weighting material, 5% to 30% by weight of biologically active agent and 5% to 15% by weight of binder material.

**Patentansprüche**

**1.** Bolus oder Pellet zur Verwendung bei der Verabreichung eines oder mehrerer aktiven Wirkstoffe an Wiederkäuer, wobei der Bolus oder das Pellet einen Körper (10, 14, 16, 20) einschließt, der aus einem aus Einzelteilen bestehenden Beschwerungsstoff besteht, dadurch gekennzeichnet, daß der Beschwerungsstoff mit einem oder mehreren biologisch aktiven Wirkstoffen gemischt wird und daß im wesentlichen alle Beschwerungsstoffpartikel eine aufgerauhte, stachelige oder unebene Oberfläche haben und zusammen mit den aktiven Wirkstoffen in einem Bindemittel dispergiert werden, so daß die Außenoberfläche des Körpers uneben ist und wie ein Schleifmittel wirkt.

**2.** Bolus oder Pellet nach Anspruch 1, worin das Bindemittel Wachs ist.

**3.** Bolus oder Pellet nach Anspruch 2, worin das Rindemittel festes Paraffin ist.

**4.** Bolus oder Pellet nach den Ansprüchen 1, 2 oder 3, worin das Bindemittel einen wasserlöslichen Wirkstoff enthält.

**5.** Bolus oder Pellet nach Anspruch 4, worin das Bindemittel Seife enthält.

**6.** Bolus oder Pellet nach Anspruch 5, worin das Bindemittel Natriumoleat enthält.

**7.** Bolus oder Pellet nach Anspruch 5, worin das Bindemittel Natriumpalmitat enthält.

**8.** Bolus oder Pellet nach Anspruch 5, worin das Bindemittel Natriumstearat enthält.

**9.** Bolus oder Pellet nach einem der vorhergehenden Ansprüche, worin der aus Einzelteilen bestehende, nichteinheitliche Beschwerungsstoff Eisen- oder Stahlschrot ist.

**10.** Bolus oder Pellet nach einem der vorhergehenden Ansprüche, worin der Körper teilweise in einem Hohlkörper eingeschlossen ist.

**11.** Bolus oder Pellet nach Anspruch 10, worin der Hohlkörper ein Rohr aus Magnesium oder Magnesiumlegierung (12, 22) ist.

**12.** Bolus oder Pellet nach Anspruch 10, worin der Hohlkörper durch eine Vielzahl von eng benachbarten Ringen (18) gebildet wird.

**13.** Bolus oder Pellet nach Anspruch 12, worin die Ringe (18) aus einem in Pansensäften nicht löslichen oder nichtabbaubaren Stoff bestehen.

**14.** Bolus oder Pellet nach Anspruch 13, worin die Ringe (18) aus Kunststoffen bestehen.

**15.** Bolus oder Pellet nach Anspruch 9, worin der Eisen-oder Stahlschrot durch Zerkleinern von kugelförmigen Granalien hergestellt wird.

16. Bolus oder Pellet nach Anspruch 1, worin der Körper (10, 14, 16, 20) 60 bis 90 Gew.-% Beschwerungsstoff, 5 bis 30 Gew.-% biologisch aktiven Wirkstoff und 5 bis 15 Gew.-% Bindemittel enthält.

**Revendications**

1. Bol ou comprimé pour utilisation dans l'administration d'un ou plusieurs agents actifs à un ruminant, ledit bol ou comprimé comprenant un corps (10, 14, 16, 20) constitué d'un matériau particulaire de lestage, caractérisé en ce que ledit matériau de lestage est mélangé avec un ou plusieurs agents biologiquement actifs, et en ce que pratiquement toutes les particules de matériau de lestage ont une surface rendue rugueuse, à pointes ou inégale, et sont dispersées, conjointement avec les agents actifs, dans un liant, de sorte que la surface externe du corps est inégale et joue le rôle d'un abrasif.

2. Bol ou comprimé selon la revendication 1, dans lequel le liant est une cire.

3. Bol ou comprimé selon la revendication 2, dans lequel le liant est la cire de paraffine.

4. Bol ou comprimé selon l'une des revendications 1, 2 et 3, caractérisé en ce que le liant comprend un agent soluble dans l'eau.

5. Bol ou comprimé selon la revendication 4, dans lequel le liant comprend un savon.

6. Bol ou comprimé selon la revendication 5, dans lequel le liant comprend de l'oléate de sodium.

7. Bol ou comprimé selon la revendication 5, dans lequel le liant comprend du palmitate de sodium.

8. Bol ou comprimé selon la revendication 5, dans lequel le liant comprend du stéarate de sodium.

9. Bol ou comprimé selon l'une quelconque des revendications précédentes, dans lequel le matériau particulaire de lestage non homogène est constitué de particules de fer ou d'acier.

10. Bol ou comprimé selon l'une quelconque des revendications précédentes, dans lequel le corps est partiellement enfermé dans un corps creux (12, 18, 22).

11. Bol ou comprimé selon la revendication 10, dans lequel le corps creux est un tube de magnésium ou d'alliage de magnésium (12, 22).

12. Bol ou comprimé selon la revendication 10, dans lequel le corps creux est muni d'une pluralité d'anneaux étroitement adjacents (18).

13. Bol ou comprimé selon la revendication 12, dans lequel les anneaux (18) sont en un matériau insoluble ou non dégradable dans les sucs de rumen.

14. Bol ou comprimé selon la revendication 13, dans lequel les anneaux (18) sont en matière plastique.

15. Bol ou comprimé selon la revendication 9, dans lequel les particules de fer ou d'acier sont produites par broyage de grenaille sphérique.

16. Bol ou comprimé selon la revendication 1, dans lequel le corps (10, 14, 16, 20) comprend de 60 à 90 % en poids de matériau de lestage, de 5 à 30 % en poids d'agent biologiquement actif et de 5 à 15 % en poids de liant.

Fig.1

Fig.2

Fig. 3

Fig. 4